# EUROPÄISCHE PATENTSCHRIFT

(11) **EP 3 658 201 B1**
(45) Veröffentlichungstag und Bekanntmachung des Hinweises auf die Patenterteilung: **26.02.2025**
(21) Anmeldenummer: 18746140.5
(22) Anmeldetag: 23.07.2018
(51) Int. Cl.: A61M 1/14, A61M 1/16, A61M 1/34, A61M 1/36

(54) **CONTAINERHALTER FÜR DIALYSEVORRICHTUNG**
CONTAINER HOLDER FOR DIALYSIS APPARATUS
SUPPORT DE RÉCIPIENT POUR DISPOSITIF DE DIALYSE

(30) Priorität: 27.07.2017 DE 102017117070
(43) Veröffentlichungstag der Anmeldung: 03.06.2020
(73) Patentinhaber: Fresenius Medical Care Deutschland GmbH, 61352 Bad Homburg (DE)
(72) Erfinder: GERCKE, Gesa, 97422 Schweinfurt (DE); RUECKERT, Jochen, 97520 Roethlein (DE); FAULHABER, Thomas, 97493 Bergrheinfeld (DE)
(74) Vertreter: Bobbert & Partner Patentanwälte PartmbB
(86) Internationale Anmeldenummer: PCT/EP2018/069900
(87) Internationale Veröffentlichungsnummer: WO 2019/020558

(56) Entgegenhaltungen:
- WO-A1-2017/019640
- WO-A1-2017/093235
- WO-A1-2018/022169
- WO-A1-2018/022170

## Beschreibung

Die vorliegende Erfindung betrifft einen Containerhalter gemäß Anspruch 1. Sie betrifft zudem eine Vorrichtung gemäß Anspruch 11.

Aus der Praxis sind Vorrichtungen zur therapeutischen Apherese, zur Leberunterstützungstherapie, zur Oxygenation, zur Peritonealdialyse oder zur Hämofiltration oder zur Hämodialyse bekannt. Sie können zur anstehenden Behandlung des Patienten mit Containern verbunden werden, die bei der Behandlung verwendete Flüssigkeiten aufweisen. Im Folgenden werden Vorrichtungen der vorstehend genannten Art zusammenfassend als Dialysevorrichtungen bezeichnet.

Aus der WO 2017/019640 A1 ist ein *Infusate Caddy* zum Tragen, Organisieren und Bedienen von Infusionsbehältern für die Dialyse bekannt.

In der WO 2017/093235 A1 sind ein medizinischer Fluidbeutelhalter und Verfahren zu seiner Herstellung, eine medizinische Wiegevorrichtung und Behandlungsvorrichtung offenbart.

Aus der WO 2018/022169 A1 ist ein Infusionshalter bekannt.

In der WO 2018/022170 A1 ist ein Rahmen für Infusionsbehälter offenbart.

Eine Aufgabe der vorliegenden Erfindung ist es, einen Halter zum Halten von derartigen Containern (kurz: Containerhalter) an Vorrichtungen sowie eine mit wenigstens einem solchen Containerhalter verbundene Vorrichtung vorzuschlagen.

Die erfindungsgemäße Aufgabe wird durch einen Containerhalter mit den Merkmalen des Anspruchs 1 gelöst. Sie wird ferner gelöst durch eine Vorrichtung mit den Merkmalen des Anspruchs 11.

Die vorliegende Erfindung betrifft einen Containerhalter für eine Dialysevorrichtung. Der erfindungsgemäße Containerhalter umfasst eine erste Containeraufnahme, welche zum Aufnehmen eines ersten Containers ausgestaltet ist, sowie eine zweite Containeraufnahme zum Aufnehmen eines zweiten Containers.

Optional weist der Containerhalter nur die erste Containeraufnahme oder nur die zweite Containeraufnahme, nicht aber weitere Containeraufnahmen, auf.

Der Containerhalter weist einen Befestigungsabschnitt zu seinem beweglichen Befestigen oder zu seiner beweglichen Befestigung an der Dialysevorrichtung auf.

Die bewegliche Befestigung kann optional in Form einer Klappvorrichtung, Verschiebevorrichtung und/oder einer lösbaren Befestigungsvorrichtung vorliegen oder durch diese gewährleistet sein; unter einer beweglichen Befestigung kann in einigen Ausführungsformen daher auch eine lösbare Befestigung verstanden werden, in anderen ist sie nicht lösbar.

Eine bewegliche Befestigung kann optional eine Relativbewegung zwischen dem Containerhalter und der Dialysevorrichtung erlauben.

Eine lösbare Befestigung kann optional dergestalt sein, dass der Containerhalter ohne Gebrauch von Werkzeug von der Dialysevorrichtung entfernbar ist und vorzugsweise ohne Werkzeug auch wieder an der Dialysevorrichtung angeordnet werden kann.

Die vorliegende Erfindung betrifft außerdem eine Dialysevorrichtung. Diese weist einen erfindungsgemäßen Containerhalter auf.

Die erfindungsgemäße Dialysevorrichtung umfasst eine Aufnahme, welche zu ihrer beweglichen Verbindung mit einem Befestigungsabschnitt des Containerhalters an einem Halteabschnitt der Dialysevorrichtung ausgestaltet ist.

Die Vorrichtung (synonym: ein entsprechender Mechanismus, beide Begriffe sind austauschbar und von der vorliegenden Erfindung umfasst) zum Klappen, Verschieben oder Lösen kann dabei durch entsprechende Vorrichtungen an entweder der Dialysevorrichtung oder dem Containerhalter oder an beiden Vorrichtungen, nämlich der Dialysevorrichtung und dem Containerhalter, vorgesehen sein.

Die Verbindung zwischen einem Hauptkörper der Dialysevorrichtung und dem Containerhalter kann auf der Rückseite des Hauptkörpers vorgesehen sein, wobei die Vorderseite eine Vielzahl (also wenigstens zwei) an Bedienungselementen für eine Behandlung aufweist. An der Vorderseite können beispielsweise eine Blutpumpe, eine Klemme für einen - bei Betrieb blutführenden - Schlauch und/oder Halterungen für einen - bei Betrieb blutführenden -Schlauch und/oder für eine - bei Betrieb blutführenden - Kassette angeordnet sein.

Bei allen hierin gemachten Ausführungen ist der Gebrauch des Ausdrucks "kann sein" bzw. "kann haben" usw. synonym zu "ist vorzugsweise" bzw. "hat vorzugsweise" usw. zu verstehen und soll Ausführungsformen erläutern.

Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt.

Ausführungsformen sind ferner Gegenstand der Unteransprüche und Ausführungsformen.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin von einer Ausführungsform die Rede ist, so handelt es sich um eine beispielhafte, erfindungsgemäße Ausführungsform.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

In einigen Ausführungsformen ist der Containerhalter als Tablett ausgestaltet, z. B. als Tablett mit genau einer Vertiefung, was seine Oberfläche betrifft.

In einigen Ausführungsformen ist der Containerhalter breiter und/oder tiefer als hoch.

Erfindungsgemäß weist der Containerhalter wenigstens eine Trennwand zwischen der ersten Containeraufnahme und der zweiten Containeraufnahme auf.

Die Trennwand, oder Abschnitte hiervon, können gerade, mit einem Krümmungsradius oder mehreren Krümmungsradien (z. B. bei Betrachtung eines Querschnitts der Trennwand) verlaufen.

Eine Trennwand entsteht in manchen Ausführungsformen dadurch, dass die erste Containeraufnahme und die zweite Containeraufnahme auf unterschiedlichen Niveaus (etwa bezogen auf die Aufstellfläche der Dialysevorrichtung im Gebrauch) angeordnet sind; die hierdurch entstehende Wand, welche die niedriger angeordnete Containeraufnahme zumindest abschnittsweise als Seitenwand begrenzt, kann als Trennwand im Sinne dieser Beschreibung verstanden werden.

Eine Trennwand entsteht in manchen Ausführungsformen dadurch, dass sich zwischen der ersten Containeraufnahme und der zweiten Containeraufnahme ein Wall, ein Wulst oder eine andere Erhebung erstreckt.

Erfindungsgemäß kann Flüssigkeit, die aus einem Container, angeordnet in der ersten Containeraufnahme, austritt, durch die "trennend" angeordnete Trennwand von der zweiten Containeraufnahme weggeleitet werden oder abgehalten werden, in diese hineinzulaufen, oder umgekehrt.

Die Containeraufnahme kann genau oder wenigstens zwei solcher Trennwände - jeweils eine für die erste und eine für die zweite Containeraufnahme - aufweisen. Die Trennwand oder die Trennwände können einen ringförmigen Querschnitt haben. Sie können einen geschlossenen Querschnitt (etwa in Form eines Rings) oder einen offenen Querschnitt (etwa in Form eines U) haben.

Die Trennwand, oder Abschnitte hiervon, können geschwungen verlaufen. Unter einer geschwungen verlaufenden Trennwand versteht man eine Trennwand, deren Höhe nicht konstant ist. Der geschwungene Verlauf kann vorteilhaft dazu beitragen, Material einzusparen.

In manchen Ausführungsformen weist die erste Containeraufnahme eine Auffangwanne auf oder besteht hieraus. Eine solche Auffangwanne dient dem Auffangen von Flüssigkeit, welche aus dem ersten Container ausgelaufen ist oder stammt, wenn dieser in der ersten Containeraufnahme platziert ist.

In einigen Ausführungsformen weist die zweite Containeraufnahme eine Auffangwanne auf oder besteht hieraus.

Eine solche Auffangwanne dient dem Auffangen von Flüssigkeit, welche aus dem zweiten Container ausgelaufen ist oder stammt, wenn dieser in der zweiten Containeraufnahme platziert ist.

In manchen Ausführungsformen umfasst der Containerhalter eine erste Aufstellfläche der ersten Containeraufnahme sowie eine zweite Aufstellfläche der zweiten Containeraufnahme. Die erste Aufstellfläche, oder Abschnitte hiervon, welche zum Aufstellen oder zum Aufnehmen des ersten Containers dient, ist im Gebrauch niedriger angeordnet als die zweite Aufstellfläche, oder Abschnitte hiervon, welche zum Aufstellen oder zum Aufnehmen des zweiten Containers dient.

In manchen Ausführungsformen sind die Aufstellfläche, oder Teile hiervon, der ersten Containeraufnahme und/oder die Aufstellfläche, oder Abschnitte hiervon, der zweiten Containeraufnahme im Gebrauch des Containerhalters niedriger angeordnet als ein Verbindungsabschnitt, welcher zwischen der ersten und/oder der zweiten Containeraufnahme verläuft und beide Containeraufnahmen miteinander verbindet.

Erfindungsgemäß umfasst die erste Containeraufnahme des Containerhalters einen (im Gebrauch) oberen Rand. Dieser obere Rand, oder Abschnitte hiervon, ist höher angeordnet als die Aufstellfläche, oder Abschnitte hiervon, der zweiten Containeraufnahme, die dem Aufstellen des zweiten Containers dient.

Die - beispielsweise bei der Desinfektion der Dialysevorrichtung eingesetzten - Container stehen mit ihren Bodenflächen (unteren Seiten) auf den Aufstellflächen oder in den Aufstellwannen der ersten und der zweiten Containeraufnahme.

Dabei können die bei der Dialyse eingesetzten Container rechteckige oder kreisförmige Bodenflächen (oder Querschnitte, gilt auch unten) (alternativ: Grundflächen) aufweisen. Die Grundformen der Aufstellflächen und/oder der Auffangwannen können an diese Formen angepasst sein.

Insbesondere kann der Containerhalter eine rechteckige erste Aufstellfläche und/oder Auffangwanne für einen Container mit rechteckiger Bodenfläche und eine kreisförmige zweite Aufstellfläche und/oder Auffangwanne für einen Container mit kreisförmiger Bodenfläche aufweisen.

Alternativ kann der Containerhalter eine rechteckige erste Aufstellfläche und/oder Auffangwanne für einen Container mit rechteckiger Bodenfläche und eine rechteckige zweite Aufstellfläche und/oder Auffangwanne für einen Container mit rechteckiger Bodenfläche aufweisen.

Weiter alternativ kann der Containerhalter eine kreisförmige erste Aufstellfläche und/oder Auffangwanne für einen Container mit kreisförmiger Bodenfläche und eine kreisförmige zweite Aufstellfläche und/oder Auffangwanne für einen Container mit kreisförmiger Bodenfläche aufweisen, oder umgekehrt.

In einigen Ausführungsformen umfasst der Befestigungsabschnitt eine oder mehrere Öffnungen. Die Öffnungen können dem Aufnehmen von Stiften, Druckknöpfen oder Sicherungszapfen dienen.

In manchen Ausführungsformen umfassen die Öffnungen des Befestigungsabschnitts wenigstens eine kreisrunde Öffnung und/oder wenigstens eine schlüssellochförmige oder sich verjüngende Öffnung.

In einigen Ausführungsformen weisen die erste Containeraufnahme und/oder die zweite Containeraufnahme eine freie Öffnung oder ein freies Inneres mit einem kreisförmigen Querschnitt zum Aufnehmen eines Containers auf.

In manchen Ausführungsformen umfasst die Dialysevorrichtung eine Aufnahme, welche wenigstens einen Stift oder einen Sicherungszapfen aufweist.

In einigen Ausführungsformen weist die Aufnahme der Dialysevorrichtung wenigstens einen Druckknopf auf.

In manchen Ausführungsformen ist die Dialysevorrichtung (auch: Dialysiervorrichtung) als Ultrafiltrationsvorrichtung, Hämofiltrationsvorrichtung, Hämodialysevorrichtung und/oder Hämodiafiltrationsvorrichtung ausgestaltet.

Die Dialysevorrichtung kann optional eine oder mehrere der folgenden Komponenten aufweisen oder hiermit verbunden sein: arterieller Leitungsabschnitt, venöser Leitungsabschnitt, arterielle Konnektionsnadel, venöse Konnektionsnadel, arterielle Patientenschlauchklemme, venöse Patientenschlauchklemme, Blutpumpe, Blutfilter, arterieller Drucksensor, venöse Tropfkammer, venöser Drucksensor, Substituatpumpe, Substituatflüssigkeitsvorratseinrichtung, Substituatflüssigkeitszuleitung, Prädilutions-Zugabestelle, Postdilutions-Zugabestelle, Signalverarbeitungseinheit, Steuerleitung zur Blutpumpe, und/oder Steuerleitung zur Substituatpumpe.

In einigen Ausführungsformen weist der Containerhalter keine beweglichen Teile auf.

In manchen Ausführungsformen ist der Containerhalter nicht Teil eines Blutkreislaufs und/oder hat keine Strömungskanäle, insbesondere keine mit geschlossenem Querschnitt und/oder ist nicht mit einem im Gebrauch fluidführenden Schlauch oder Kanal verbunden und/oder weist keine arterielle und/oder venöse Blutkammer auf und/oder weist keine im Gebrauch von einen Fluid durchflossen beweglichen Teile auf.

In einigen Ausführungsformen weist der Containerhalter nicht sowohl eine obere als auch eine untere Hälfte oder einen Grundkörper und eine Abdeckung hierfür, welche zum Gebrauch zusammengesetzt werden, auf.

In manchen Ausführungsformen ist der Containerhalter einstückig.

In einigen Ausführungsformen ist der Containerhalter integral gefertigt, etwa im Spritzgussverfahren.

In einigen Ausführungsformen weist der Containerhalter Aussparungen oder unterschiedliche Dicken auf, was dem Ziel der Gewichts- und Materialeinsparung dienen kann.

In einigen Ausführungsformen ist der Containerhalter mehrstückig. So kann er z. B. ein im Spritzgussverfahren gefertigtes Teil, z. B. ein Oberteil, aufweisen, welches mit einem z. B. aus Metall gefertigten Teil, z. B. einem Unterteil, verbunden ist oder hierauf ruht. Eine solche Kombination von Materialien kann Vorteile aufweisen, die die hohe Festigkeit von Metall und die günstige Fertigung von Spritzteilen umfassen können.

In einigen Ausführungsformen ist die erste Containeraufnahme oder die zweite Containeraufnahme eine, vorzugsweise plane, Aufstellfläche.

In einigen Ausführungsformen ist die erste Containeraufnahme oder die zweite Containeraufnahme nur geeignet und/oder vorgesehen, auf einer Seite hiervon (vorzugsweise auf der Oberseite, nicht auch auf der Unterseite) einen Container aufzunehmen oder zu berühren.

In einigen Ausführungsformen weisen der Containerhalter, die erste Containeraufnahme und/oder die zweite Containeraufnahme nur auf einer Seite hiervon (vorzugsweise auf der Oberseite, nicht auch auf der Unterseite) eine Öffnung zum Einführen eines Containers, oder eines Abschnitts hiervon, auf.

In einigen Ausführungsformen weist der Containerhalter nur eine Öffnung zum Einführen eines Containers, oder eines Abschnitts hiervon, auf.

In einigen Ausführungsformen weist der Containerhalter nur einen wannenartigen Abschnitt (etwa eine Auffangwanne), insbesondere zum Auffangen von Flüssigkeiten, auf.

In einigen Ausführungsformen weisen die erste Containeraufnahme und die zweite Containeraufnahme unterschiedlich hohe (die Höhe wird z. B. im Gebrauch, z. B. vom Boden, auf welchem die die Containeraufnahme tragende Dialysevorrichtung steht, gemessen) Aufstellflächen für den jeweiligen Container oder unterschiedlich hohe Bodenflächen auf.

In einigen Ausführungsformen weist der Containerhalter keine freistehenden und/oder aufrecht stehenden Säulen und/oder kein absorbierendes Material auf.

In einigen Ausführungsformen ist der Containerhalter kein Einwegartikel und/oder kein modularer Artikel.

In einigen Ausführungsformen weist der Containerhalter keinen Deckel, insbesondere keinen abnehmbaren oder keinen aufgeschweißten Deckel, und/oder keine zweikanaligen Steckverbinder auf.

Erfindungsgemäß besteht der Containerhalter aus einem Thermoplast oder weist einen solchen auf. Es kann sich bei dem Thermoplast um Polyamid-6 bzw. Polycaprolactam handeln. Der Thermoplast, insbesondere das Polyamid-6, kann kugel- und/oder faserverstärkt sein, etwa durch Glas, Carbon oder andere Materialien. Der Thermoplast kann medienbeständig sein.

Die Aufnahme und der Befestigungsabschnitt können zusammen eine Rastverbindung ermöglichen. Sie können eine selbsthemmende Verbindung ermöglichen, die insbesondere winkelabhängig ist. Sie können eine Hinterschneidung aufweisen und/oder einen Eingriff einer dieser beiden Komponenten in die andere dieser beiden Komponenten bewirken.

Die Aufnahme und der Befestigungsabschnitt können zusammen wenigstens eine sich verjüngende Öffnung aufweisen. Sie können eine Pilzkopfverriegelung aufweisen.

In einigen Ausführungsformen weist der Containerhalter einen ersten Container und einen zweiten Container auf. Optional weist er neben diesen beiden Containern keinen weiteren Container auf.

In einigen Ausführungsformen weist der erste Container eine Flüssigkeit aus der Gruppe der sauren Lösungen, insbesondere der sauren Desinfektionsmittel, auf. Diese sind dadurch gekennzeichnet, dass sie einen pH-Wert kleiner als 7 aufweisen.

Die saure Lösung kann reduzierbare Ionen enthalten, beispielsweise Chlorid-Ionen.

Die Flüssigkeit des ersten Containers kann eine einzelne Säure oder eine Mischung aus verschiedenen Säuren aufweisen, insbesondere aus der Gruppe, welche aus Salzsäure, Essigsäure, Zitronensäure, Milchsäure, Apfelsäure besteht.

Bei der Flüssigkeit des ersten Containers kann es sich um eine Mischung aus Zitronensäure, beispielsweise in Form des Monohydrats, Apfelsäure und Milchsäure handeln.

Bei der Flüssigkeit des ersten Containers kann es sich um das Desinfektionsmittel, wie es unter dem Markennamen Citrosteril von Fresenius Medical Care vertrieben wird, handeln.

Der diese Flüssigkeit enthaltende Container kann eine rechteckige Bodenfläche (oder Querschnitte) aufweisen.

In einigen Ausführungsformen weist der zweite Container eine Flüssigkeit aus der Gruppe der oxidierenden Lösungen, insbesondere der oxidierenden Desinfektionsmittel, auf. Diese sind dadurch gekennzeichnet, dass zumindest ein Bestandteil eine oxidierende Wirkung auf eine säurehaltige Lösung aufweist. Beispiele für solche säurehaltigen Lösungen sind in den vorangehenden Abschnitten genannt. Die oxidierende Lösung kann eine oder mehrere oder alle der Substanzen Hypochlorit, Peroxid, Persäure aufweisen.

Bei der Flüssigkeit des zweiten Containers kann es sich um eine wässrige Lösung von Natriumhypochlorit, Kaliumhydroxid und/oder Natriumhydroxid handeln.

Bei der Flüssigkeit des zweiten Containers kann es sich um das Desinfektionsmittel, wie es unter dem Markennamen Sporotal von Fresenius Medical Care vertrieben wird, handeln.

Die Flüssigkeit des ersten und/oder zweiten Containers ist optional kein Infusat, keine Dialysierflüssigkeit, kein Dialysat, kein Effluent und/oder kein Substituat, oder Vorstufen hiervon.

Der diese Flüssigkeit enthaltende Container kann eine kreisförmige Bodenfläche aufweisen.

Beim Entnehmen der Flüssigkeiten aus den Containern kann es zu einem unkontrollierten Austritt der Flüssigkeiten aus den Container kommen, beispielsweise durch Herausschwappen, Anhaften der Flüssigkeit an einer Entnahmevorrichtung, Überlaufen. Dadurch können die beiden Flüssigkeiten in Kontakt miteinander kommen. Hierdurch kann es zu einer Gefährdung kommen. Das Oxidationsmittel kann mit der sauren Lösung reagieren. Eine solche Reaktion kann wie folgt erfolgen, wobei giftiges Chlorgas freigesetzt wird:

2H₃0+ + OCl⁻ + Cl⁻ -> 3H₂0 + Cl₂

In einigen Ausführungsformen weist der Containerhalter kein - insbesondere relativ zur einer der Aufstellflächen oder Containeraufnahmen - bewegbares Element auf, insbesondere keinen bewegbaren Befestigungsabschnitt und/oder kein bewegbares Befestigungsmittel.

In einigen Ausführungsformen weist der Containerhalter keinen Henkel und/oder keinen vom übrigen Containerhalter abstehenden Griffabschnitt auf.

In einigen Ausführungsformen weist der Containerhalter keine Einrichtung auf, mit welcher es sich direkt (also nicht mittels der Dialysevorrichtung) am Boden abstützt, wenn er mit der Dialysevorrichtung verbunden ist, insbesondere keine Rollen.

In einigen Ausführungsformen weist der Containerhalter keine Einrichtung auf, mit welcher er in einer Daten-, Signal-, Fluid- und/oder Energieübertragungsverbindung mit der Dialysevorrichtung steht.

In einigen Ausführungsformen stehen der Containerhalter und/oder die auf ihm angeordneten Container nicht in einer, beispielsweisen starren, Fluidübertragungsverbindung mit der Dialysevorrichtung. In einigen Ausführungsformen kann lediglich eine über einen flexiblen Schlauch herstellbare Fluidübertragungsverbindung vorgesehen sein.

In einigen Ausführungsformen ist der Befestigungsabschnitt, welcher der Verbindung des Containerhalters mit der Aufnahme der Dialysevorrichtung dient, mit - vorzugsweise nur - einer der Containeraufnahmen, welche zum Aufnehmen jeweils eines Containers dienen, verbunden oder dieser zugeordnet. Diese Containeraufnahme kann die erste Containeraufnahme sein. Sie kann die größte aller vorhandenen Containeraufnahme sein. Sie kann eine Containeraufnahme sein, welche keine Vertiefung aufweist. Sie kann jene Containeraufnahme sein, die im Gebrauchszustand näher zur Aufnahme der Dialysevorrichtung steht oder ohne Spalt zur Aufnahme angeordnet ist. Hierdurch kann jeweils eine höhere Stabilität für das Anordnen vergleichsweise großer Container erzielt werden. Zudem kann gewährleistet sein, dass eine aus einem der Container austretende Flüssigkeit zumindest über Abschnitte des Containerhalters, an welchen dieser vorzugsweise keinen Befestigungsabschnitt hat, zwischen Dialysevorrichtung und Containerhalter ablaufen kann, ohne das Gehäuse der Dialysevorrichtung zu benetzen.

In einigen Ausführungsformen ist der Befestigungsabschnitt, welcher der Verbindung des Containerhalters mit der Aufnahme der Dialysevorrichtung dient, angeordnet, um mit einem unteren oder mittleren Abschnitt, nicht aber mit einem oberen Abschnitt, der Aufnahme verbunden zu werden.

Auf diese Weise steht in manchen Ausführungsformen zumindest der obere Abschnitt der Aufnahme über dem Befestigungsabschnitt, über wenigstens einer Containeraufnahme oder dem gesamten Containerhalter nach oben über. Hierdurch kann gewährleistet sein, dass eine aus einem der Container austretende Flüssigkeit nur die Aufnahme, nicht aber das Gehäuse der Dialysevorrichtung oder Komponenten der Dialysevorrichtung (wie Anschlüssen, usw.) benetzt. Die Aufnahme, welche eine vergleichsweise kleine Komponente ist, kann aus Material gefertigt sein, welches sich für einen Kontakt mit austretender Flüssigkeit besonders eignet.

In einigen Ausführungsformen beträgt die Breite des Containerhalters zwischen 30 cm und 50cm. Dies erlaubt es, zwei Container nebeneinander auf dem Containerhalter aufzustellen.

In einigen Ausführungsformen beträgt die Tiefe des Containerhalters zwischen 14 cm und 25cm, vorzugsweise zwischen 17 cm und 20 cm. Dies erlaubt es, die Tiefe eines Containers bequem aufzunehmen.

In einigen Ausführungsformen beträgt die Tiefe wenigstens einer der Containeraufnahmen (vom Boden bis zum oberen Rand) wenigstens 8 cm, bevorzugt 9 cm, 10 cm, 11 cm oder mehr. Dies erlaubt es, z. B. einen 1-Liter-Container kippsicher in der Containeraufnahme aufzubewahren.

In einigen Ausführungsformen weist der Containerhalter wenigstens eine ebene Fläche mit einer Breite von wenigstens 15 cm, bevorzugt wenigstens 17 cm, und einer Tiefe von wenigstens 15 cm, bevorzugt wenigstens 17 cm, auf. Dies erlaubt es, Container bequem aufzunehmen, welche ein Fassungsvermögen von mehr als einem Liter haben.

Manche oder alle erfindungsgemäßen Ausführungsformen können einen, mehrere oder alle der oben und/oder im Folgenden genannten Vorteile aufweisen.

Ein mittels bestimmter erfindungsgemäßer Ausführungsformen erzielbarer Vorteil besteht darin, dass Flüssigkeiten wie z. B. Desinfektionsmittel, die aus den auf dem Containerhalter aufgestellten Containern verschüttet werden oder überlaufen, nicht miteinander in Kontakt geraten. Chemische Reaktionen zwischen ihnen können so vorteilhaft vermieden werden. Hierzu können die o. g. Trennwand, die Auffangwanne, und weitere hierin genannte Merkmale beitragen.

Von Vorteil kann ferner sein, dass aufgrund der niedrigen Anordnung des Halteabschnitts an der Dialysevorrichtung, deren Stabilität durch die Anordnung der Container nicht verschlechtert wird, was sich insbesondere beim rollenden Fortbewegen der Dialysevorrichtung angenehm bemerkbar machen kann.

Vorgesehene Trennwände können dem auf dem Containerhalter aufgestellten Container ferner eine seitliche Stütze geben. Diese können hierdurch vor einem Umfallen, etwa beim Fortbewegen der Dialysevorrichtung, geschützt sein.

Aufgrund der beweglichen Befestigung kann es vorzugsweise möglich sein, den Containerhalter platzsparend für beispielsweise den Transport - etwa an der Dialysevorrichtung - anzuordnen oder von dieser abzunehmen.

Der Befestigungsabschnitt für die bewegliche Befestigung des Containerhalters, insbesondere in Form eines manuell abnehmerbaren oder lösbaren Befestigungsabschnitts, kann ein einfaches Reinigen des Containerhalters und/oder der Dialysevorrichtung erlauben.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:
- Fig. 1: zeigt einen erfindungsgemäßen Containerhalter in einer ersten exemplarischen Ausführungsform;
- Fig. 2: zeigt den erfindungsgemäßen Containerhalter der Fig. 1 in einer anderen Perspektive;
- Fig. 3: zeigt eine erfindungsgemäße Dialysevorrichtung;
- Fig. 4a, 4b: zeigen den Containerhalter und die Aufnahme der vorangegangenen Figuren im Schnitt in einem Gebrauchszustand (Fig. 4a) bzw. während seines Verbindens mit oder Lösens von der Dialysevorrichtung;
- Fig. 5: zeigt eine beispielhafte Ausgestaltung des erfindungsgemäßen Containerhalters in einer zweiten Ausführungsform; und
- Fig. 6: zeigt den Containerhalter der Fig. 5 in einem Schnitt.

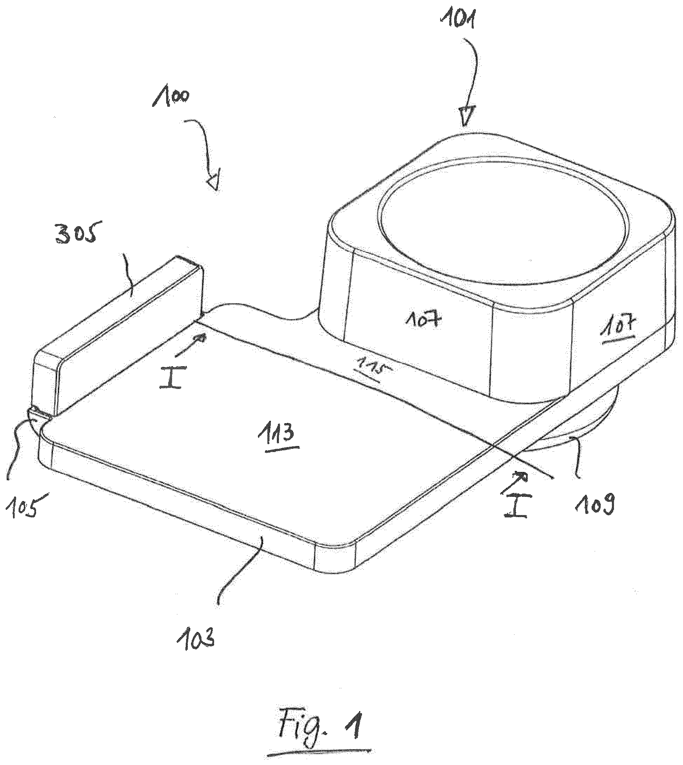

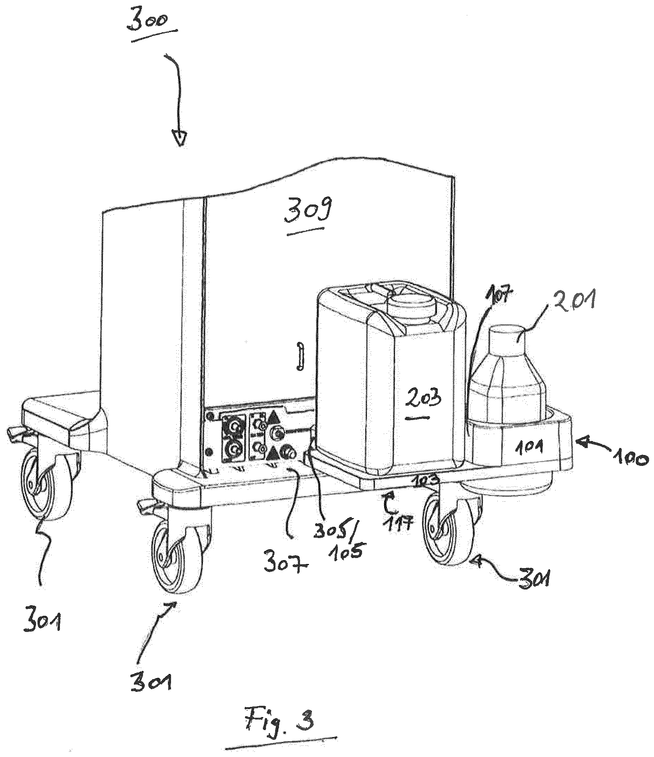

**Fig. 1** zeigt eine erste Ausführungsform des erfindungsgemäßen Containerhalters 100. Er weist eine erste Containeraufnahme 101 zum Aufnehmen eines ersten Containers (in Fig. 1 nicht gezeigt, siehe jedoch das Bezugszeichen 201 in Fig. 3) und eine zweite Containeraufnahme 103 zum Aufnehmen eines zweiten Containers (in Fig. 1 nicht gezeigt, siehe jedoch das Bezugszeichen 203 in Fig. 3) auf.

Der Containerhalter 100 weist ferner einen Befestigungsabschnitt 105 zum beweglichen, hier lösbaren, Befestigen des Containerhalters 100 an einer Dialysevorrichtung auf (in Fig. 1 nicht gezeigt, siehe jedoch das Bezugszeichen 300 in Fig. 3). Der Befestigungsabschnitt 105 ist zur Verbindung mit einer Aufnahme 305 der Dialysevorrichtung 300 ausgestaltet. Er dient der lösbaren Verbindung des Containerhalters 100 an einem Halteabschnitt der Dialysevorrichtung 300.

Die erste Containeraufnahme 101 ist als ein von einer Trennwand 107 umgebener, nach oben offener Raum, hierin auch als ein Inneres bezeichnet, ausgestaltet.

Die Trennwand 107 kann in jeder Ausführungsform umlaufend, also geschlossen sein. Es können alternativ mehrere Trennwände 107 vorgesehen sein, die unter Abstand zueinander angeordnet sind.

Trennwände 107 können insbesondere zwischen der ersten Containeraufnahme 101 und der zweiten Containeraufnahme 103 vorgesehen sein.

Trennwände 107 können derart verlaufen, dass Flüssigkeiten, die sich auf dem Boden einer der Containeraufnahmen 101, 103 befinden, daran gehindert werden, in die jeweils andere Containeraufnahme zu fließen.

Der Raum kann, wie in Fig. 1 optional gezeigt, mittels eines Bodenabschnitts 109 nach unten begrenzt sein. Er kann mittels der Aufstellfläche nach unten ebenfalls geschlossen sein, so dass Flüssigkeiten den Raum nicht nach unten verlassen können. Der Bodenabschnitts 109 kann als Auffangwanne ausgestaltet sein.

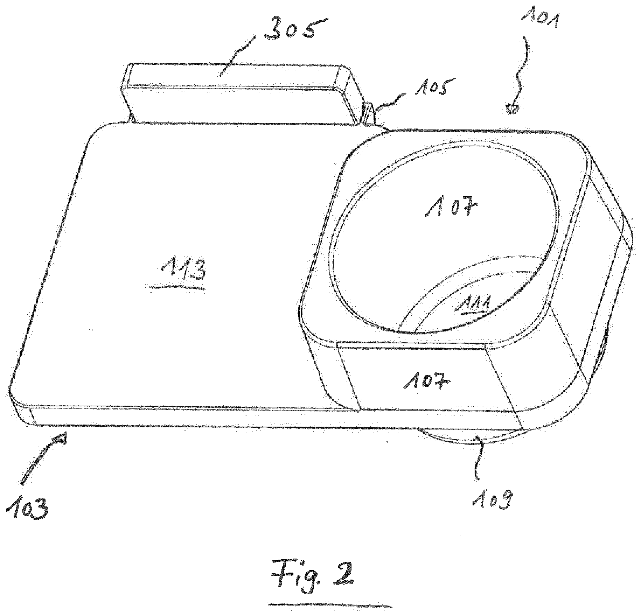

Der Bodenabschnitt 109 kann an seiner Oberseite eine erste Aufstellfläche 111 (zu sehen in Fig. 2) zum Aufstellen oder zum Aufnehmen des ersten Containers 201 aufweisen. Die erste Aufstellfläche 111 kann planar oder gekrümmt sein. Sie kann frei von Erhebungen sein. Sie kann eine Durchgangsöffnung mit einem Verschlussmittel aufweisen.

Die zweite Containeraufnahme 103 weist eine Oberseite auf, welche zum Aufstellen oder zum Aufnehmen des in Fig. 1 nicht gezeigten zweiten Containers 203 dient. Die Oberseite wird daher als zweite Aufstellfläche 113 bezeichnet. Sie kann planar oder gekrümmt sein. Sie kann frei von Erhebungen sein. Sie kann eine Durchgangsöffnung mit einem Verschlussmittel aufweisen.

Die erste Containeraufnahme 101 und die zweite Containeraufnahme 103 sind durch einen Verbindungsabschnitt 115 miteinander verbunden. Der Verbindungsabschnitt 115 liegt zwischen ihnen. Er kann nahtlos in die zweite Containeraufnahme 103 übergehen.

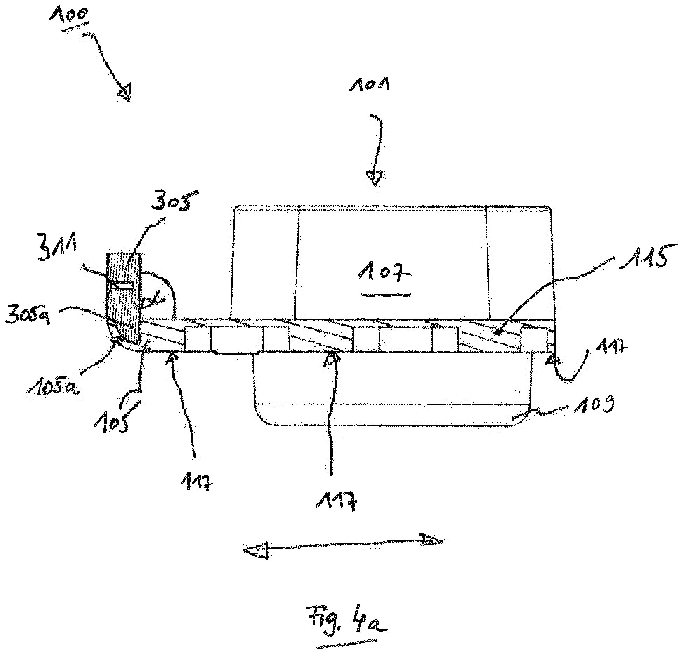

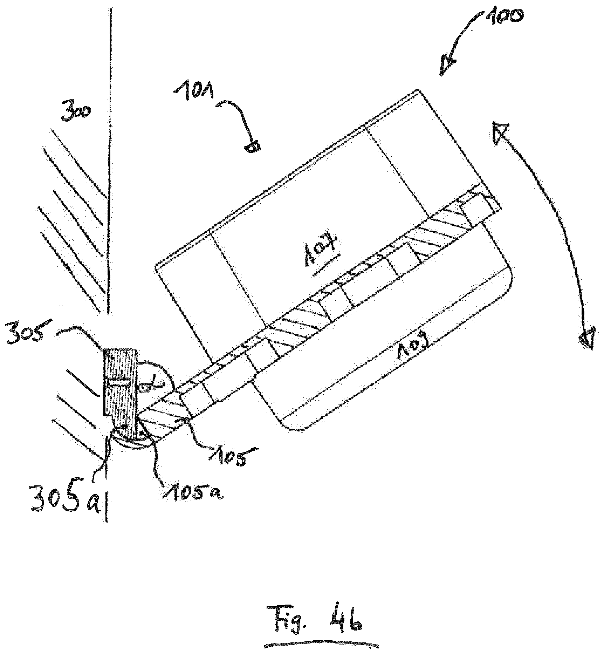

Die lösbare Verbindung zwischen Befestigungsabschnitt 105 und Aufnahme 305 ist in Fig. 4a und 4b im Detail gezeigt.

Der Befestigungsabschnitt 105 und/oder die Aufnahme 305 können jeweils einstückig sein.

Der Befestigungsabschnitt 105 und/oder die Aufnahme 305 können jeweils eine Länge von mehreren Zentimetern aufweisen. Aufgrund ihrer Länge verteilen sich mechanische Belastungen, die sich aus der Verbindung von Befestigungsabschnitt 105 und Aufnahme 305 ergeben können, auf eine entsprechend große Fläche.

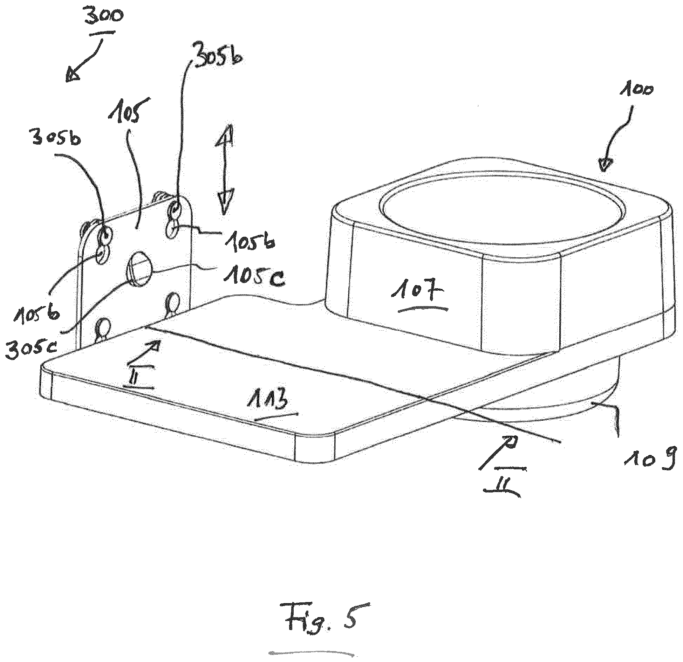

Anders als in Fig. 1 (und Fig. 5) gezeigt, können eine Vielzahl von Befestigungsabschnitten 105 und/oder Aufnahmen 305 vorgesehen sein.

**Fig. 2** zeigt den Containerhalter 100 der Fig. 1 in anderer Perspektive.

Die Breite des Containerhalters 100 erstreckt sich in der Rechts-Links-Richtung von Fig. 2. Die Tiefe erstreckt sich (leicht perspektivisch verzerrt) von oben nach unten in Fig. 2. Die Dicke des Containerhalters 100 kann im Bereich der ersten Containeraufnahme 101 vorzugsweise zwischen 1 cm und 2 cm betragen.

**Fig. 3** zeigt eine erfindungsgemäße Dialysevorrichtung 300 von der Rückseite in leichter Perspektive von oben.

Die Dialysevorrichtung 300 weist optional Rollen 301 auf, z. B. vier Stück. Sie dienen der Fortbewegung der Dialysevorrichtung 300 und können optional eine Arretiervorrichtung aufweisen.

Die Dialysevorrichtung 300 weist an ihrer Rückseite einen Halteabschnitt auf, an welchem die Aufnahme 305 angeordnet ist.

Der Halteabschnitt liegt im Beispiel der Fig. 3 in einem bodenseitigen Bereich der Dialysevorrichtung 300, z. B. wenig oberhalb der Rollen 301.

Der Halteabschnitt kann optional so niedrig angeordnet sein, dass eine Unterseite 117 der zweiten Containeraufnahme 103 eine Oberfläche 307 der Dialysevorrichtung 300 oder seines Halteabschnitts berührt und/oder sich auf dieser abstützt. Letzteres kann vorteilhaft der sicheren und stabilen Anordnung des Containerhalters 100 an der Dialysevorrichtung 300 dienen. Zudem erlaubt eine solche Abstützung die Verwendung entsprechend kleiner dimensionierter und kostengünstigerer Komponenten wie Befestigungsabschnitt 105 oder Aufnahme 305.

Der Halteabschnitt kann der Befestigung der Rollen 301 an der restlichen Dialysevorrichtung 300 dienen.

Der Halteabschnitt kann eine sog. Medienschiene sein, also ein Abschnitt, an welchem sich auch ein oder mehrere Wasserzuläufe, ein oder mehrere Gaszugänge und/oder ein oder mehrere Anschlüsse für eine Stromversorgung befinden. Die Medienschiene kann ein Interface der Hydraulik zu externen Anschlüssen sein. Sie kann die Aufgabe haben, Wasser und/oder Reinigungsmittel in die Dialysevorrichtung einzuleiten, Dialyseflüssigkeit zum Patienten (Substitution) oder zum Dialysator zu leiten und Flüssigkeit vom Dialysator sowie Dampf aus der Maschine auszuleiten.

Wie Fig. 3 zeigt, kann der Containerhalter 100 derart am Halteabschnitt der Dialysevorrichtung 300 befestigt sein, dass nur die zweite Containeraufnahme 103 auf dem Halteabschnitt ruht und/oder von einer Rückwand 309 der Dialysevorrichtung 300 bedeckt wird oder hinter dieser liegt, während die erste Containeraufnahme 101 seitlich über die Dialysevorrichtung 300 oder deren Rückwand 309 hervorsteht. Dadurch kann optional erreicht werden, dass andere Zugänge freiliegen. Diese Zugänge können beispielsweise Teil der Medienschiene sein. Dadurch kann optional auch erreicht werden, dass eine oder mehr Mediumansaugvorrichtungen, die an der Vorderseite der Dialysevorrichtung 300 angeordnet sind und mittels derer über Schlauchleitungen der Inhalt aus den Containern in die Dialysevorrichtung 300 saugbar sind, keine so langen Schläuche benötigen. Der seitliche Versatz kann optional ferner eine Kollision des Containers mit Komponenten an der Rückwand der Dialysevorrichtung 300 verhindern. Er kann optional erlauben, dass die Container von der Vorderseite der Dialysevorrichtung 300 aus sichtbar und halbwegs zugänglich sind und/oder, ggf. eine Kollision der Trennwand 107 im hochgeklappten Zustand mit der Rückwand der Dialysevorrichtung 300 verhindern. Hierdurch kann auch eine größere Freiheit bei der Festlegung einer in Querrichtung des Containerhalters 100 verlaufenden Tiefe der ersten Containeraufnahme 101 gegeben sein. Die Tiefe verläuft in Richtung des in Fig. 4a gezeigten Doppelpfeils. Die Tiefe der erste Containeraufnahme 101 kann, wie in Fig. 3 gezeigt, größer sein als die Tiefe der zweiten Containeraufnahme 103. Sie kann jedoch auch gleich oder geringer als jene sein.

**Fig. 4a** **und** **Fig. 4b** zeigen den Containerhalter 100 und die Aufnahme 305 der vorangegangenen Figuren von der Seite, in einem Schnitt durch den Verbindungsabschnitt 115, den Befestigungsabschnitt 105 und die Aufnahme 305, entlang der Linie I-I der Fig. 1, also mit Blick auf die nicht geschnittene erste Containeraufnahme 101. Container 201 oder 203 sind nicht dargestellt.

Fig. 4a zeigt den Befestigungsabschnitt 105 und die Aufnahme 305 in einem verrasteten oder vollständig verbundenen Zustand, wie er im Gebrauchszustand vorliegt und daher optional als Gebrauchszustand verstanden werden kann.

Dabei greift ein endseitiger Abschnitt 305a der Aufnahme 305, der als eine Nase ausgeformt sein kann, in eine Aussparung 105a, die von einer Oberseite des Befestigungsabschnitts 105 ausgehend vorgesehen sein kann, ein. Dieser, oder ein anders ausgestalteter kraft- und/oder formschlüssiger Eingriff der Aufnahme 305 in den Befestigungsabschnitt 105, legt den Containerhalter 100 zumindest in einer Richtung fest, die in der Haupterstreckungsebene des Containerhalters 100 liegt. In Fig. 4a ist diese Richtung eine Rechts-Links-Richtung und mittels Doppelpfeils bezeichnet.

Wie Fig. 4a zu entnehmen ist, kann der endseitige Abschnitt 305a der Aufnahme 305 durch eine Stufe von weiteren Abschnitten der Aufnahme 305 abgesetzt sein. Er kann eine unter einem Radius zulaufende Hinterseite haben. Er kann eine gerade verlaufende Vorderseite haben.

Der endseitige Abschnitt 305a der Aufnahme 305 kann zumindest an seiner Stirnseite und ggf. auch an Seitenabschnitten jeweils von Flächen der Aussparung 105a berührt werden oder in Kontakt stehen.

Die Aufnahme 305 kann vorbereitet sein, um mit dem Halteabschnitt der Dialysevorrichtung 300 verschraubt zu werden. Hierzu kann sie eine oder mehrere Öffnungen, z. B. Gewindeöffnungen 311, aufweisen. Diese Öffnungen können Durchgangsöffnungen oder, wie in Fig. 4a exemplarisch gezeigt, Sacköffnungen sein. Im Beispiel der Fig. 4a wird die Aufnahme 305 von ihrer hinteren Seite, also von der den Containeraufnahmen 101, 103 abgewandten Seite, verschraubt. Andere lösbare oder unlösbare Verbindungen zwischen

Aufnahme 305 und Halteabschnitt sind ebenfalls von der vorliegenden Erfindung umfasst.

Ein Winkel α zwischen Befestigungsabschnitt 105 und Aufnahme 305 beträgt im Gebrauchszustand beispielsweise zwischen 80° und 100°, etwa 90° oder einen rechten Winkel.

Die Aufnahme 305 kann senkrecht zu einer Aufstellebene der Dialysevorrichtung 300 und/oder zu einer Haupterstreckungsebene des Containerhalters 100 in dessen Gebrauchszustand stehen.

Fig. 4b zeigt den Befestigungsabschnitt 105 und die Aufnahme 305 in einem nicht-verrasteten oder nichtvollständig verbundenen Zustand. Fig. 4b zeigt nicht den Gebrauchszustand des Containerhalters 100. Sie zeigt vielmehr eine Stellung desselben, die dieser bei seinem Verbinden mit der Dialysevorrichtung 300 oder bei seinem Lösen von dieser einnimmt.

Der Winkel α beträgt weniger als 80°, hier etwa 45°. Er muss zum abschließenden Verbinden des Containerhalters 100 an der Dialysevorrichtung 300 auf das in Fig. 4a gezeigte Winkelmaß vergrößert werden.

Wie Fig. 4b zu entnehmen ist, erfolgt das Verbinden des Containerhalters 100 mit der Dialysevorrichtung 300 oder sein Lösen von dieser unter Schwenken des Containerhalters 100 in einer in Fig. 4b mittels Doppelpfeils gezeigten Richtung.

Das Drehzentrum der vorstehend genannten Schwenkbewegung liegt, vereinfacht angenommen, im endseitigen Abschnitt 305a, oder nahe diesem.

**Fig. 5** zeigt den erfindungsgemäßen Containerhalter 100 in einer zweiten exemplarischen Ausführungsform.

Der Containerhalter 100 der Fig. 5 entspricht mit Ausnahme seines Befestigungsabschnitts 105 dem Containerhalter 100 der ersten Ausführungsform. Der Befestigungsabschnitt 105 ist im Beispiel der Fig. 5 anders als im Beispiel der Fig. 4a/Fig. 4b nicht ausgestaltet, um durch Schwenken mit der Aufnahme 305 verbunden zu werden. Vielmehr wird der Befestigungsabschnitt 105 durch sein Verschieben von oben nach unten (bezogen auf Fig. 5) mit der Aufnahme 305 verbunden. Um ihn von der Aufnahme 305 zu trennen, muss er (jedenfalls auch) nach oben (bezogen auf Fig. 5) geschoben werden. Der Doppelpfeil der Fig. 5 deutet dies jeweils an.

Wie Fig. 5 zeigt, weist der Befestigungsabschnitt 105 wenigstens eine Öffnung 105b auf, in welche ein Stift 305b der Aufnahme 305 eingreifen kann. Die Öffnung 105b ist als sich nach oben verjüngende Öffnung ausgestaltet. Sie kann die Form eines (umgedrehten) Schlüssellochs haben.

Der Stift 305b weist endseitig eine Verbreiterung auf. Er ist exemplarisch mit einer Pilzkopfform ausgestaltet. Mit der Verbreiterung passt er durch den nicht-verjüngten Abschnitt der Öffnung 105b, nicht jedoch durch deren verjüngten Abschnitt. Auf diese Weise kann eine Verriegelung zwischen Stift 305b und Öffnung 105b auf bekannte Weise erzielt werden.

Die Öffnung 105b kann wie in Fig. 5 exemplarisch gezeigt als Durchgangsöffnung ausgestaltet sein, kann aber auch eine Sacklochöffnung sein.

Fig. 5 zeigt vier Öffnungen 105b. Mehr oder weniger Öffnungen dieser Art sind ebenfalls von der Erfindung umfasst. Dasselbe gilt für die Anzahl der Stifte 305b.

Fig. 5 zeigt ferner eine optionale Öffnung 105c des Befestigungsabschnitts 105, die vorzugsweise als Durchgangsöffnung ausgestaltet ist. Die Öffnung 105c dient der Aufnahme eines Druckstifts, eines Druckbolzens oder eines Druckknopfs 305c, welcher jeweils vorrichtungsseitig vorgesehen ist.

Anders als die Öffnungen 105b (bezogen auf die Stifte 305b) ist die Öffnung 105c nicht ausgestaltet, um sich zum Trennen von Druckknopf 305c und Öffnung 105c relativ zum Druckknopf 305c verschieben zu lassen. Vielmehr soll die Öffnung 105c ein Verschieben des Befestigungsabschnitts 105 relativ zur Aufnahme 305 gerade verhindern, solange der Druckknopf 305c sich in oder durch die Öffnung 105c erstreckt. Damit verhindert der in der Öffnung 105c steckende Druckknopf 305c, dass der Befestigungsabschnitt 105 ungewollt aus der Aufnahme 305 gelöst werden kann, etwa durch einen Tritt mit dem Fuß gegen die Unterseite des Containerhalters 100, und sich dieser unbeabsichtigt von der Dialysevorrichtung 300 löst.

Zum Lösen des Containerhalters 100 von der Dialysevorrichtung 300 ist zunächst der Druckknopf 305c zu drücken, mit dem Ziel, ihn - ggf. gegen einen Widerstand, der nur beispielsweise durch eine Federeinrichtung erzeugt werden kann - aus der Öffnung 105c herauszudrücken. Erst wenn er außer Eingriff zur Öffnung 105c ist, kann der Containerhalter 100 durch Verschieben nach oben von den Stiften 305b und damit von der Dialysevorrichtung 300 gelöst werden.

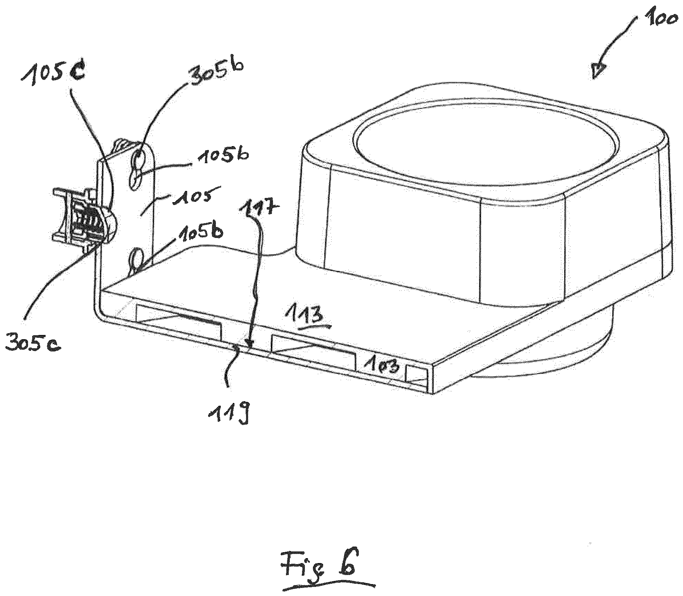

**Fig. 6** zeigt den Containerhalter 100 der Fig. 5 in einem Schnitt entlang der Linie II-II der Fig. 5.

Wie zu erkennen ist, setzt sich der Befestigungsabschnitt 105 in einer Bodenplatte 119 fort, auf welcher die zweite Containeraufnahme 103, die getrennt von der Bodenplatte 119 gefertigt wurde, mit ihrer Unterseite 117 angeordnet ist oder ruht.

Der Fachmann erkennt, dass es für die angestrebte Funktion unerheblich ist, ob die Stifte 305b und/oder der Druckknopf 305c auf Seite der Dialysevorrichtung 300 und die entsprechenden Öffnungen 105b bzw. 105c auf der Seite des Containerhalters 100 vorliegen, oder umgekehrt. Dasselbe gilt für die Komponenten 105 und 305 der in Fig. 1 gezeigten Ausführungsform. Auch sie können jeweils alternativ auf dem Containerhalter 100 oder auf der Dialysevorrichtung 300 vorliegen.

Wie den Figuren zu entnehmen ist, kann der Containerhalter Abschnitte mit unterschiedlicher Tiefe aufweisen, was nicht zuletzt in einer Draufsicht, siehe z. B. Fig. 2, die einer Draufsicht sehr nahe kommt, zu erkennen ist.

### Bezugszeichenliste

- 100: Containerhalter
- 101: erste Containeraufnahme
- 103: zweite Containeraufnahme
- 105: Befestigungsabschnitt
- 105a: Aussparung
- 105b: Öffnung
- 105c: (optionale) Öffnung
- 107: Trennwand
- 109: Bodenabschnitt
- 111: erste Aufstellfläche
- 113: zweite Aufstellfläche
- 115: Verbindungsabschnitt
- 117: Unterseite
- 119: Bodenplatte

- 201: erster Container
- 203: zweiter Container

- 300: Dialysevorrichtung
- 301: Rollen
- 305: Aufnahme
- 305a: endseitiger Abschnitt
- 305b: Stift
- 305c: Druckknopf
- 307: Oberfläche
- 309: Rückwand
- 311: Gewindeöffnung

- α: Winkel zwischen Befestigungsabschnitt und Aufnahme

## Patentansprüche

1. Containerhalter (100) bestehend aus einem Thermoplast oder einen solchen aufweisend für eine Vorrichtung, welche eine Dialysevorrichtung (300), insbesondere eine Ultrafiltrationsvorrichtung, Hämofiltrationsvorrichtung, Hämodialysevorrichtung und/oder Hämodiafiltrationsvorrichtung, oder eine Vorrichtung zur therapeutischen Apherese, zur Leberunterstützungstherapie oder zur Oxygenation ist, mit wenigstens
- einer ersten Containeraufnahme (101) zum Aufnehmen eines ersten Containers (201);
- einer zweiten Containeraufnahme (103) zum Aufnehmen eines zweiten Containers (203);
- einem Befestigungsabschnitt (105) zum beweglichen Befestigen des Containerhalters (100) an der Vorrichtung;
wobei zwischen der ersten Containeraufnahme (101) und der zweiten Containeraufnahme (103) wenigstens eine Trennwand (107) angeordnet ist,
**dadurch gekennzeichnet, dass** die Trennwand (7) angeordnet ist, um Flüssigkeit, die aus einem Container, angeordnet in der ersten Containeraufnahme, austritt, davon abzuhalten, in die zweite Containeraufnahme hineinzulaufen, oder umgekehrt.

2. Containerhalter (100) nach Anspruch 1, wobei die erste Containeraufnahme (101) eine Auffangwanne zum Auffangen von aus dem ersten Container (201), wenn dieser in der ersten Containeraufnahme (101) platziert ist, auslaufender oder stammender Flüssigkeit, aufweist oder hieraus besteht.

3. Containerhalter (100) nach einem der vorangehenden Ansprüche, wobei die zweite Containeraufnahme (103) eine Auffangwanne zum Auffangen von aus dem zweiten Container (203), wenn dieser in der zweiten Containeraufnahme (103) platziert ist, auslaufender oder stammender Flüssigkeit, aufweist oder hieraus besteht.

4. Containerhalter (100) nach einem der vorangehenden Ansprüche, wobei eine erste Aufstellfläche (111), oder Abschnitte hiervon, der ersten Containeraufnahme (101) zum Aufstellen oder zum Aufnehmen des ersten Containers (201) im Gebrauch des Containerhalters (100) niedriger angeordnet ist als eine zweite Aufstellfläche (113), oder Abschnitte hiervon, der zweiten Containeraufnahme (103) zum Aufstellen oder zum Aufnehmen des zweiten Containers (203).

5. Containerhalter (100) nach einem der vorangehenden Ansprüche, wobei die erste Aufstellfläche (111), oder Abschnitte hiervon, der ersten Containeraufnahme (101) und/oder die zweite Aufstellfläche (113), oder Abschnitte hiervon, der zweiten Containeraufnahme (103) im Gebrauch des Containerhalters (100) niedriger angeordnet ist als ein Verbindungsabschnitt (115) zwischen der ersten Containeraufnahme (101) und/oder der zweiten Containeraufnahme (103).

6. Containerhalter (100) nach einem der vorangehenden Ansprüche, wobei ein oberer Rand, oder Abschnitte hiervon, der ersten Containeraufnahme (101) im Gebrauch des Containerhalters (100) höher angeordnet ist als die zweite Aufstellfläche (113), oder Abschnitte hiervon, der zweiten Containeraufnahme (103) zum Aufstellen oder zum Aufnehmen des zweiten Containers (203).

7. Containerhalter (100) nach einem der vorangehenden Ansprüche, wobei der Befestigungsabschnitt (105) eine Klappvorrichtung, eine Verschiebevorrichtung und/oder ein lösbares Befestigungsmittel aufweist.

8. Containerhalter (100) nach einem der vorangehenden Ansprüche, wobei der Befestigungsabschnitt (105) eine oder mehrere Öffnungen (105b) zum Aufnehmen von Stiften (305b), Druckknöpfen (305c) oder Sicherungszapfen aufweist.

9. Containerhalter (100) nach Anspruch 8, wobei die Öffnungen wenigstens eine kreisrunde Öffnung (105c) und/oder wenigstens eine schlüssellochförmige oder sich verjüngende Öffnung (105b) umfassen.

10. Containerhalter (100) nach einem der vorangehenden Ansprüche, wobei die erste Containeraufnahme (101) und/oder die zweite Containeraufnahme (103) eine Öffnung oder ein freies Inneres mit einem kreisförmigen Querschnitt aufweisen.

11. Vorrichtung, welche eine Dialysevorrichtung (300), insbesondere eine Ultrafiltrationsvorrichtung, Hämofiltrationsvorrichtung, Hämodialysevorrichtung und/oder Hämodiafiltrationsvorrichtung, oder eine Vorrichtung zur therapeutischen Apherese, zur Leberunterstützungstherapie oder zur Oxygenation ist, **gekennzeichnet durch** wenigstens
- einen Containerhalter (100) nach einem der Ansprüche 1 bis 10; und
- eine Aufnahme (305), ausgestaltet zu ihrer beweglichen, insbesondere klappbaren, verschiebbaren oder lösbaren, Verbindung mit dem Befestigungsabschnitt (105) des Containerhalters (100) zum Befestigen des Containerhalters (100) an einem Halteabschnitt der Vorrichtung.

12. Vorrichtung nach Anspruch 11, wobei die Aufnahme (305) wenigstens einen Stift (305b) oder Sicherungszapfen aufweist.

13. Vorrichtung nach einem der Ansprüche 11 bis 12, wobei die Aufnahme (305) wenigstens einen Druckknopf (305c) aufweist.

## Claims

1. A container holder (100) consisting of a thermoplastic or comprising such one, for an apparatus, which is a dialysis apparatus (300), in particular an ultrafiltration apparatus, hemofiltration apparatus, hemodialysis apparatus and/or hemodialysis apparatus, or an apparatus for therapeutic apheresis, liver support therapy or oxygenation, with at least:
- a first container receptacle (101) for receiving a first container (201);
- a second container receptacle (103) for receiving a second container (203);
- a fastening section (105) for movably fastening the container holder (100) to the apparatus;
wherein at least one partition wall (107) is arranged between the first container receptacle (101) and the second container receptacle (103),
**characterized in that**, the partition wall (107) is arranged to prevent liquid leaking from a container arranged in the first container receptacle from flowing into the second container receptacle or vice versa.

2. The container holder (100) according to claim 1, wherein the first container receptacle (101) comprises or consists of a drip pan for collecting liquid flowing from or originating from the first container (201), when the latter is placed in the first container receptacle (101).

3. The container holder (100) according to anyone of the preceding claims, wherein the second container receptacle (103) comprises or consists of a drip pan for collecting liquid flowing from or originating from the second container (203), when the latter is placed in the second container receptacle (103).

4. The container holder (100) according to anyone of the preceding claims, wherein a first positioning surface (111), or sections thereof, of the first container receptacle (101) for positioning or receiving the first container (201), is arranged, during use of the container holder (100), lower than a second positioning surface (113), or sections thereof, of the second container receptacle (103) for positioning or receiving the second container (203).

5. The container holder (100) according to anyone of the preceding claims, wherein the first positioning surface (111), or sections thereof, of the first container receptacle (101) and/or the second positioning surface (113), or sections thereof, of the second container receptacle (103) is arranged, during use of the container holder (100), lower than a connecting section (115) between the first container receptacle (101) and/or the second container receptacle (103).

6. The container holder (100) according to anyone of the preceding claims, wherein an upper edge, or sections thereof, of the first container receptacle (101) is arranged, during use of the container holder (100), higher than the second positioning surface (113), or sections thereof, of the second container receptacle (103) for positioning or receiving the second container (203).

7. The container holder (100) according to anyone of the preceding claims, wherein the fastening section (105) comprises a folding device, a shifting device and/or a releasable fastening means.

8. The container holder (100) according to anyone of the preceding claims, wherein the fastening section (105) comprises one or several openings (105b) for receiving pins (305b), push buttons (305c) or securing pins.

9. The container holder (100) according to claim 8, wherein the openings comprise at least one round opening (105c) and/or at least one keyhole-shaped or tapered opening (105b).

10. The container holder (100) according to anyone of the preceding claims, wherein the first container receptacle (101) and/or the second container receptacle (103) comprise(s) an opening or a free interior with an circular cross-section.

11. An apparatus which is a dialysis apparatus (300), in particular an ultrafiltration apparatus, hemofiltration apparatus, hemodialysis apparatus and/or hemodiafiltration apparatus, or an apparatus for therapeutic apheresis, for liver support therapy or for oxygenation, **characterized by** at least:
- one container holder (100) according to anyone of the claims 1 to 10; and
- one receptacle (305) designed for its movable, in particular foldable, shiftable or releasable, connection with the fastening section (105) of the container holder (100) in order to fasten the container holder (100) to a holding section of the apparatus.

12. The apparatus according to claim 11, wherein the receptacle (305) comprises at least one pin (305b) or securing pin.

13. The apparatus according to anyone of the claims 11 to 12, wherein the receptacle (305) comprises at least one push button (305c).

## Revendications

1. Un support de conteneur (100) constitué d'un thermoplastique ou comprenant un tel thermoplastique, pour un appareil, qui est un appareil de dialyse (300), notamment un appareil d'ultrafiltration, un appareil d'hémofiltration, un appareil d'hémodialyse et/ou un appareil d'hémodiafiltration, ou un appareil d'aphérèse thérapeutique, de thérapie de soutien hépatique ou d'oxygénation, comportant au moins :
- un premier réceptacle de conteneur (101) pour accueillir un premier conteneur (201);
- un second réceptacle de conteneur (103) pour accueillir un second conteneur (203);
- une section de fixation (105) pour fixer de manière mobile le support de conteneur (100) à l'appareil;
où au moins une cloison (107) est agencée entre le premier réceptacle de conteneur (101) et le second réceptacle de conteneur (103),
**caractérisé en ce que** la cloison (107) est agencée pour empêcher un liquide sortant d'un conteneur agencé dans le premier réceptacle de conteneur de s'écouler dans le second réceptacle de conteneur ou vice versa.

2. Le support de conteneur (100) selon la première revendication, où le premier réceptacle de conteneur (101) comprend ou est constitué d'un bac de récupération pour recueillir un liquide s'écoulant ou provenant du premier réceptacle de conteneur (201), lorsque ce dernier est placé dans le premier réceptacle de conteneur (101).

3. Le support de conteneur (100) selon l'une quelconque des revendications précédentes, où le second réceptacle de conteneur (103) comprend ou est constitué d'un bac de récupération pour recueillir un liquide s'écoulant ou provenant du second réceptacle de conteneur (203), lorsque ce dernier est placé dans le second réceptacle de conteneur (103).

4. Le support de conteneur (100) selon l'une quelconque des revendications précédentes, où une première surface de positionnement (111), ou des parties de celle-ci, du premier réceptacle de conteneur (101), destinée à positionner ou à accueillir le premier conteneur (201), est disposée plus bas, lors de l'utilisation du support de conteneur (100), qu'une seconde surface de positionnement (113), ou des parties de celle-ci, du second réceptacle de conteneur (103), destinée à positionner ou à accueillir le second conteneur (203).

5. Le support de conteneur (100) selon l'une quelconque des revendications précédentes, où la première surface de positionnement (111), ou des parties de celle-ci, du premier réceptacle de conteneur (101) et/ou la seconde surface de positionnement (113), ou des parties de celle-ci, du second réceptacle de conteneur (103), est/sont disposée(s) plus bas, lors de l'utilisation du support de conteneur (100), qu'une partie de raccordement (115) entre le premier réceptacle de conteneur (101) et/ou le second réceptacle de conteneur (103).

6. Le support de conteneur (100) selon l'une quelconque des revendications précédentes, où un bord supérieur, ou des parties de celui-ci, du premier réceptacle de conteneur (101) est disposé plus haut, lors de l'utilisation du support de conteneur (100), que la seconde surface de positionnement (113), ou des parties de celle-ci, du second réceptacle de conteneur (103) destiné à positionner ou à accueillir le second conteneur (203).

7. Le support de conteneur (100) selon l'une quelconque des revendications précédentes, où la section de fixation (105) comporte un dispositif de pliage, un dispositif de déplacement et/ou un moyen de fixation amovible.

8. Le support de conteneur (100) selon l'une quelconque des revendications précédentes, où la section de fixation (105) comporte une ou plusieurs ouvertures (105b) pour accueillir des goupilles (305b), des boutons-pression (305c) ou des goupilles de sécurité.

9. Le support de conteneur (100) selon la revendication 8, où les ouvertures comportent au moins une ouverture circulaire (105c) et/ou au moins une ouverture en forme de trou de serrure ou une ouverture conique (105b).

10. Le support de conteneur (100) selon l'une quelconque des revendications précédentes, où le premier réceptacle de conteneur (101) et/ou le second réceptacle de conteneur (103) comporte(nt) une ouverture ou un intérieur libre présentant une section transversale circulaire.

11. Un appareil, qui est un appareil de dialyse (300), notamment un appareil d'ultrafiltration, un appareil d'hémofiltration, un appareil d'hémodialyse et/ou un appareil d'hémodiafiltration, ou un appareil d'aphérèse thérapeutique, de thérapie de soutien hépatique ou d'oxygénation, **caractérisé par** au moins :
- un support de conteneur (100) selon l'une quelconque des revendications 1 à 10; et
- un réceptacle (305), conçu pour son raccordement mobile, notamment pliable, déplaçable ou amovible, avec la section de fixation (105) du support de conteneur (100) afin de fixer le support de conteneur (100) à une section de maintien de l'appareil.

12. L'appareil selon la revendication 11, où le réceptacle (305) comporte au moins une goupille (305b) ou une goupille de sécurité.

13. L'appareil selon l'une quelconque des revendications 11 à 12, où le réceptacle (305) comporte au moins un bouton-pression (305c).
